# EUROPEAN PATENT SPECIFICATION

(11) **EP 1 110 605 B1**
(45) Date of publication and mention of the grant of the patent: **26.11.2008**
(21) Application number: 00308126.2
(22) Date of filing: 18.09.2000
(51) Int. Cl.: B01J 35/04, B01J 37/02, C07C 17/02, C07C 17/154, C07C 17/156, C07C 17/10

(54) **Use of a metallic monolitihic catalyst support for selective gas phase reactions**
Verwendung einer monolitischen metallischen Katalysatorträger zur selektiven Gasphasenreaktionen
Utilisation d'un support de catalyseur monolithique métallique pour les réactions sélectives

(30) Priority: 24.12.1999 GB 9930666; 29.12.1999 GB 9930751
(43) Date of publication of application: 27.06.2001
(73) Proprietor: Ineos Vinyls UK Ltd., Runcorn Cheshire WA7 4JE (GB)
(72) Inventor: Carmello, Diego, 31021 Mogliani Veneto (TV) (IT); Marsella, Andrea, 31038 Paese (TV) (IT); Forzatti, Pio, 20052 Monza (MI) (IT); Tronconi, Enrico, 20043 Arcore (MI) (IT); Groppi, Gianpiero, 20043 Segrate (MI) (IT)
(74) Representative: King, Alex

(56) References cited:
- GB-A- 1 492 929
- US-A- 4 888 320
- US-A- 5 099 085
- US-A- 5 208 206
- US-A- 5 986 152

## Description

The present invention relates to monolith catalyst supports for selective gas phase exothermic reactions carried out in fixed bed tubular reactors. In particular the present invention refers to the use of metallic monolith catalyst supports in selective gas-phase exothermic reactions, which use shows improved selectivity and increased catalyst lifetime combined with very low pressure drop along the reactor.

It is well known that gas phase exothermic catalytic reactions present a number of difficulties when performed in large reactors on an industrial scale. The main problem is how to dissipate the heat released as the reagents are converted into products.

In the prior art it is known that fluid bed reactors have been developed in order to overcome that problem. However they have serious drawbacks, e.g. difficulty in scaling up from the pilot stage to an industrial reactor, catalyst losses due to carry over from the reactors, and low selectivity due to the back mixing regime of the fluid bed. Moreover sometimes it is not possible to have the catalyst suitable for a specific reaction in powder form and with such mechanical properties as are appropriate for a fluid bed reactor.

It is also known from the prior art how to carry out exothermic reactions recycled in fixed bed reactors packed with catalysts in pellet form. However hot spots are present in the catalytic bed, as it is difficult to remove the reaction heat. Hot spots lead to catalyst deactivation and to a decrease in selectivity. As a further disadvantage of the packed bed reactor, the pressure drop is typically very large across the length of the reactor. In an industrial plant such a pressure drop requires that the reacting gases should be compressed to high pressure. This involves a high energy consumption. Further, the unreacted gases have to be compressed before they are used in the reactor.

The prior art does not teach how to carry out an exothermic reaction in a fixed bed reactor operated in a nearly isothermal mode, and achieving high selectivity and very low pressure drop. In industrial practice, in order to reduce the hot spot temperatures with the pellet catalyst beds, a series of complicated measures must be adopted, including narrow tubes, distributed feeds along the reactor, multiple reactors, and multiple layers of catalysts with different activities and different levels of inert dilutions. All of these measures result in more expensive industrial plants and complex process operations; in any case the hot spot temperatures remain high.

Operation of endothermic reactions in packed-bed tubular catalytic reactors also involves difficulties associated with the efficient and uniform transport of the heat of reaction from the reactor tube walls to the catalytically active material, resulting in the formation of cold spots.

In order to overcome the above disadvantages, USP 5,099,085 describes the use of honeycomb monolithic catalyst supports (instead of conventional pellets) for exothermic selective chlorination and/or oxychlorination reactions in multitubular reactors with a fixed-bed arrangement of the catalyst. The materials used for preparing the monolith support are activated alumina, aluminium silicate, silica gel, titanium oxide, silicon carbide or mixtures of said materials, or sintered ceramics such a α-Al₂O₃. Mullite and cordierite are preferred. With the ceramic monolith supports described in this patent and with the related process engineering measures it is possible to reduce the pressure drop, to suppress as far as possible the formation of hot spots and to increase the selectivity in the target products. The ceramic monolith supports have a length from a few centimeters up to about 20 cm and the cross-sectional size, corresponding to the diameter of the reactor tubes, is usually smaller than 20 to 50 mm. The individual catalyst modules (monoliths) are spaced from each other by glass spheres, having a diameter of 3 to 6 mm. From the industrial point of view the spherical packing for spacing makes difficult and complicated the loading of the reactor tubes with the monoliths. In addition, the short monolith length makes the loading operation even more difficult. A further disadvantage of the process of said patent resides in the fact that the hot spot is reduced in respect to the traditional fixed bed catalyst, but it is still too high. In order to obtain this reduced hot spot the reacting gases have to be introduced at different points of the reactor (see Fig. 1 of said patent) thereby creating a multireactor system in order to get better thermal control. Experiments carried out by the present Applicant using the monoliths according to said US patent in a single reactor and feeding the reactants at one point, i.e. using a single reactor, have shown that the hot spot is too high and the selectivity too low for an industrial application (see comparative example).

US 5,208,206 relates to "an exhaust gas purifying catalyst". Similarly, US 4,888,320 relates to "a catalyst for cleaning exhaust gas from an engine". Thus, the catalysts of these documents are directed to the simultaneous reaction of various compounds i.e. the pollutants in engine exhaust gases. GB 1,492,929 relates to catalysts and catalytic structures comprising a substrate, an intermediate oxide layer and a catalytic layer, specifically disclosing a number of non-selective reactions.

The present Applicant has unexpectedly and surprisingly found a method for solving the above technical problem in fixed bed reactors by using monolith catalyst supports for selective gas-phase exothermic reactions in tubular reactors as defined hereinbelow.

The present invention provides the use of a catalyst comprising
a catalyst support that comprises a metallic monolith having channels the walls of which are adapted to receive a catalytically active phase or an intermediate layer acting as a carrier for a catalytically active phase;
wherein the intermediate layer is made of material selected from aluminum hydroxides, aluminum oxide-hydroxides, alumina, silica, zirconia, titania, magnesia, pumice, diatomaccous earth, zeolites and their mixtures; and
catalytically active material deposited on the walls of the channels or optionally on the intermediate layer,
in a selective gas-phase exothermic reaction selected from the selective chlorination and/or oxychlorination of alkenes or alkanes or the selective oxidation of alkenes.

It has been found by the present Applicant that the metallic monolith is at the same time the support of the catalytically active phase and the medium for removing efficiently the heat of reaction and substantially avoiding the hot spot of the fixed bed reactors.

The monolith is suitably provided with channels that are substantially parallel to the longitudinal axis of the monolith and of the reactor in which it is to be used.

The perpendicular cross section of the channels, hereinafter designated as "cells", is delimited by a closed line, represented by the perimeter of the cross section of the channels.

Every regular or irregular shape of the cell perimeter can be used if necessary, the preferred ones being square, triangular, hexagonal and circular, since they are easy to manufacture. The cell density, i.e. the number of cells per unit cross sectional area of the monolith, is preferably at least 3 cells/cm², more preferably between 8 and 100 cells/cm², in order to assure a sufficient geometric surface of the monolith walls. The size of the cells, defined by means of the hydraulic diameter, i.e. four times the ratio of the cross sectional area to the perimeter of the cell, is generally less than 5 mm, preferably between 1 and 3 mm. This reduced size represents an advantage since it is possible to put more cells in the monolith per unit of cross sectional area. Although the cells can be different from each other in size, a uniform size is preferred since this makes the monolith easier to manufacture.

The volume fraction of the metallic support is preferably less than 0.9, more preferably between 0.15 and 0.6. This reduced fraction of metallic support represents an advantage since it allows the maintenance of high monolith void fractions, thus further reducing pressure drops. Further, a reduced volume fraction of the support allows an important saving of expensive support material and a reduction of the reactor weight.

The geometric surface area per unit volume of the monolith should preferably be at least 6 cm²/cm³, preferably at least 10 cm²/cm³. In this way the productivity of the reactor can be increased. By "productivity" is meant the amount of the desired product(s) per unit time and per unit volume of the reactor. In addition the requested overall catalyst inventory is achieved through a lower thickness of the catalyst layer to be deposited on the metal monolith surface with respect to the ceramic monolith. This represents an advantage since the thinner is the catalytic layer, the stronger is the adhesion, and therefore its stability in the reactor is improved.

The present Applicant has found that preferably it is sufficient to fix one of the above properties in the range indicated, i.e. the values of the cell density, or of the size of the cells, or of the thickness of the channel walls, or of the surface area per unit volume of the monolith, in order to obtain the best results of the invention. More preferably two of the above four properties are fixed, still more preferably three of them, even still more preferably all four of them.

The length of the monolith, it has been found, is not particularly limited; the preferred length is usually chosen on the basis of the cost or ease of production. Generally it is between 5 cm and the total length of the reactor tubes; preferably it is in the range 30 cm - 1m.

The metallic structure formed by the channel walls of the monoliths is continuous in order to ensure substantial elimination of hot spots in exothermic reactions, and of cold spots in endothermic ones. The monolith should be in contact with the fixed bed reactor tube walls in such a way as to provide sufficient heat removal.

It has been found by the present Applicant that the substantial elimination of hot spots can be obtained by preparing the monoliths by any known technique, such as extrusion of metals or metallic powders, folding and/or stacking metallic foils or sheets, provided that the above continuity is assured. The techniques for preparation of metallic monoliths are well known. See for example X. Xu and J.A. Moulijn in "Structured Catalysts and Reactors", (A. Cybulski and J.A. Moulijn Eds.), M. Dekker, New York, 1998. The metals suitable for making the monolith are not particularly limited. We mention as preferred copper, aluminum, nickel or their alloys. Other alloys, such as FecralloyTM , may also be used. FecralloyTM is an alloy comprising predominantly iron (72.8%), chromium (22%) and aluminum (5%), with minor amounts of yttrium (0.1%) and zinc (0.1%).

The catalytic material can be deposited on the surface of the monolith by any known technique. As far as the deposition is concerned, the
- intermediate layer of material can make easier the deposition of catalytically active components and, if necessary, can avoid contact between the monolith material and the reaction environment. The material of the intermediate layer selected from aluminum hydroxides, aluminum oxide-hydroxides, alumina, silica, zirconia, titania, magnesia, pumice, diatomaceous earth, zeolites or their mixtures.

As an example can be mentioned the so called washcoating technique, consisting in the deposition of a layer of intermediate material, often with high specific surface area, onto the surface of the monolith. Washcoating is usually performed by bringing the monolith into contact with a slurry of fine powder, or with a solution containing the desired compound with optional subsequent precipitation, or with a sol of the desired material followed by gelation. Alternatively the intermediate layer can be obtained by a number of other methods such as thermal coating, electroplating, anodization, chemical or physical vapour deposition, dipping in molten materials. For the washcoating techniques see e.g. Brinker et al., "Sol-Gel Science. The physics and chemistry of sol-gel processing", Academic Press, Boston, 1990; M.F.M. Zwinkels, S.G. Jaras, P. Govind Menon in "Preparation of Catalysts VI", (G. Poncelet et al. Eds.), Elsevier, Amsterdam, 1995; P. Govind Menon, M.F.M. Zwinkels et al., Kinetics and Catalysis, 39, 670 (1998).

The catalytically active components can be incorporated into the intermediate layer during the washcoating step or after the washcoat has been deposited, using any well known technique, such as dipping, chemical vapour deposition or in situ synthesis. In some cases the catalytically active components can be deposited directly onto the monolith walls, avoiding the intermediate washcoating step.

The reactor tubes are loaded by pushing the monoliths sequentially one after the other into the tubes. Usually the channels of each monolith are aligned in order to minimize the pressure drop across the tube.

The advantages of using metallic monoliths as catalyst supports for exothermic reactions are:
- to have a flat temperature profile along the tube, substantially avoiding hot spots and performing the reaction at the proper selected temperature; in such a way it is possible to prevent or to slow down the catalyst deactivation and to increase the selectivity of the reaction;
- to adopt reactor tubes with larger diameters and so to reduce dramatically the number of tubes for the same capacity, hence allowing the cost of the plant to be reduced. The diameter of the conventional reactor tube is about 25 to 30 mm; with the monolith of the invention the diameter can reach 80 mm or even higher, preferably 50 - 80mm. On the contrary in the prior art, in order to minimize the hot spots, tubular fixed bed reactors are made up of tubes with narrow diameters. This is a disadvantage for the industrial capacity, since reactors with a large number of tubes are required;
- to achieve very low pressure drop across the tube: this pressure drop is often over one order of magnitude less than the pressure drop in conventional packed bed tubular reactors; this lower pressure drop could be exploited in processes where it is necessary to recycle back unreacted products; in this case instead of using a compressor, a blower may be used with significant energy saving;
- to carry out the exothermic reaction in one single reactor without splitting the inlet reactants along the reactor tube; this avoids the multiple feed inlets of the prior art, as stated above;
- to use higher coolant temperatures in externally cooled tubular reactors for exothermic reactions while maintaining still acceptable hot spot temperatures in the catalytic bed: accordingly, the overall catalytic activity of the bed is enhanced and therefore the required amount of catalyst load can be reduced;
- to achieve nearly isothermal conditions in fixed-bed reactors such as those obtained in fluid bed reactors, without difficulties associated with scaling up from the pilot stage to an industrial reactor, catalyst losses due to carry over from the reactors, and low selectivity due to the back mixing regime of the fluid bed.

The exothermic reactions in which the monolith of the invention can be applied are selected from the selective chlorination and/or oxychlorination of alkenes, e.g. ethylene, or alkanes, e.g. methane and ethane; and selective oxidation of alkenes, e.g. ethylene and propylene. As examples can be mentioned the conversion of ethylene with chlorine to 1,2-dichloroethane, the conversion of ethylene with hydrogen chloride and air or oxygen to give 1,2-dichloroethane, and the reaction of methane with chlorine.

The catalyst for the oxychlorination reaction of ethylene preferably contains copper, preferably in an amount of 1 to 12 wt% of the intermediate layer. Preferably the catalytically active material also comprises at least one of alkali metals, alkaline earth metals, group IIB metals and lanthanides in a total amount up to 10 wt % of the intermediate layer. Preferably the alkali metal is lithium or potassium, the alkaline earth is magnesium and the lanthanide is lanthanum or cerium, and each of them is present in an intermediate layer in an amount up to 6 wt %.

The catalytically active material for selective oxidation of alkenes contains for example silver. The catalytically active material may preferably also comprise at least one of alkali metals, alkaline earth metals, group IIB metals and lanthanides in a total amount preferably up to 5 wt % on the intermediate layer. Preferably the alkali metal is caesium, the alkaline earth is barium.

The catalyst for the oxychlorination of alkanes contains in the intermediate layer for example copper and an alkali metal in the atomic ratio 2:8. The alkali metal is preferably potassium or lithium. The catalytically active material may preferably also comprise at least one of alkaline earth metals, group IIB metals and lanthanides. Preferably the alkaline earth is magnesium and the lanthanide is lanthanum or cerium.

For the selective chlorination of alkenes and alkanes, the well known active components of the catalysts are used, for example alkali metals, alkaline earth metals, group IIB metals and lanthanides in a total amount preferably up to 30 wt % on the intermediate layer.

The following Examples refer to the oxychlorination reaction of ethylene to 1,2-dichloroethane, taken as representative of selective gas phase exothermic reactions. Such examples are given for illustrative purposes and are not intended to limit the scope of the invention.

### EXAMPLES

The accompanying drawings are for the purpose of illustrating the invention.

In the drawings,
Figure 1 comprises a schematic perspective view, not to scale, of a monolith support and catalyst as used in the invention, used in the following Examples, and a sectional view of one of the cells of the catalyst;
Figure 2 is a diagram of the apparatus used in the activity tests; and
Figures 3 to 6 are graphs illustrating the temperature profiles obtained in various test runs described below.

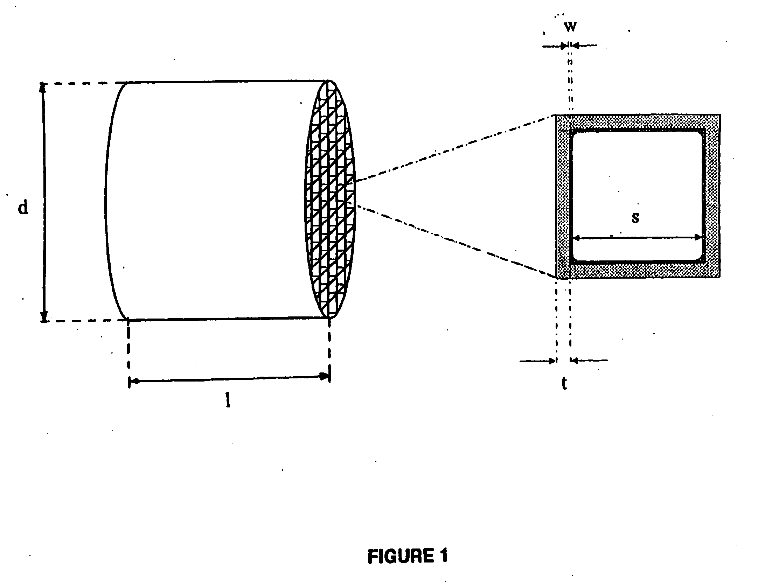

With reference to Figure 1, four sets of monolith catalysts were prepared, with supports made of different metals or alloys: Fecralloy ®, nickel, aluminum and copper.

Each monolith had a cylindrical shape with parallel channels of square cross section (see Fig. 1). The length (1) of each monolith was 50 cm, its external diameter (d) being the same as the internal diameter of the reactor tubes used for catalytic activity testing, as described later on. The side of the square cells (s) was 3.1 mm, while the wall thickness (t) was 0.43 mm.

A first layer of washcoating (carrier) was deposited by dipping into an aqueous slurry of commercial boehmite (supplied by Condea). After removal of excess liquid by blowing with air, the monolith was slowly dried under mild conditions. A layer of ?-alumina having a surface area of 190 m²/g was applied as a washcoat onto the monolith walls, using the same procedure as above, followed by flash drying at 280°C. The concentration of the slurry and the withdrawal speed were adjusted in order to obtain a total layer thickness (w) of respectively 65 µm for the use in the tube 9m long and 85 µm for the use in the tube 6.5 m long.

After calcination at increasing temperature up to 550°C, the washcoat was impregnated by dipping with an aqueous solution of the active components, i.e. copper chloride and potassium chloride, with a concentration suitable to obtain the desired concentration of the active components in the washcoat: 8% wt Cu and 0.8 % wt. K. Finally the impregnated monoliths were dried at 150°C for 4 hours. The void fraction of the so obtained catalytic bed was 0.7.

For comparative purposes a set of ceramic (cordierite) monoliths was also prepared. The ceramic monoliths had cylindrical shape with parallel channels having square cross section. The length of each monolith was 20 cm, its external diameter being the same as the internal diameter of the reactor tubes used for catalytic activity testing, as described later on. The internal side of the square cells was 0.5 mm, while the wall thickness was 0.9 mm. Although it is well known that ceramic monoliths with thinner walls can be obtained, the choice of the above parameters was determined by the necessity to keep the hot spot temperature as low as possible. In spite of that, during the activity tests it was necessary also to reduce the coolant temperature in order to moderate the hot spot temperature. Attempts to use monoliths with characteristics more similar to those of the metallic ones failed since they gave rise to unacceptable hot spot temperatures. On the other hand the above mentioned choice of parameters resulted in monoliths with low geometric surface area per unit volume, so it was necessary to increase the thickness of the washcoat layer in order to achieve a total quantity of active components per unit volume of the reactor sufficient to ensure the desired conversion values. The thickness of the washcoat layer, obtained by repeated impregnations, was 100 µm instead of 65 µm, with the same concentration of copper and potassium chlorides as the metallic monoliths. Due to the larger walls and washcoat thickness of the ceramic monoliths, the void fraction of the so obtained catalytic bed was 0.1.

The choice of the method used for catalytic activity testing is very important because the differences in terms of conversion and selectivity exhibited by different catalysts are usually small, but of great importance on an industrial scale. In order to obtain results which could be truly representative of the industrial reactor, a test was performed by using a tube having the same size as an industrial one and the same conditions (temperature, pressure, feed composition, feed flow rate) were adopted as those used in the industrial reactor. Under this point of view a part of the data reported below, concerning Fecralloy®, nickel, aluminum and copper based monoliths, was obtained in the pilot plant by using a tube having the same size as a typical industrial reactor, i.e. an inner diameter of 27.2 mm and a length of 9 m.

However, the peculiar properties of the catalyst or catalyst support of the present invention allow the use of the catalyst also in tubes having a size so large as to be unsuitable for industrial reactors operating with conventional packed beds of catalyst pellets. In order to point out other uses of the object of the present invention, the results obtained in very large tubes (low cost reactor) are shown later. The large tube chosen for the example had an internal diameter of 56.0 mm and a length of 9 m.

A further advantage of the catalyst of the present invention is that a very high conversion is obtained even in reactors equipped with tubes shorter than the conventional ones, simply by increasing the washcoat layer thickness. To demonstrate this, a few test runs were performed in a large tube (56.0 mm internal diameter) with a length of 6.5 m instead of 9 m.

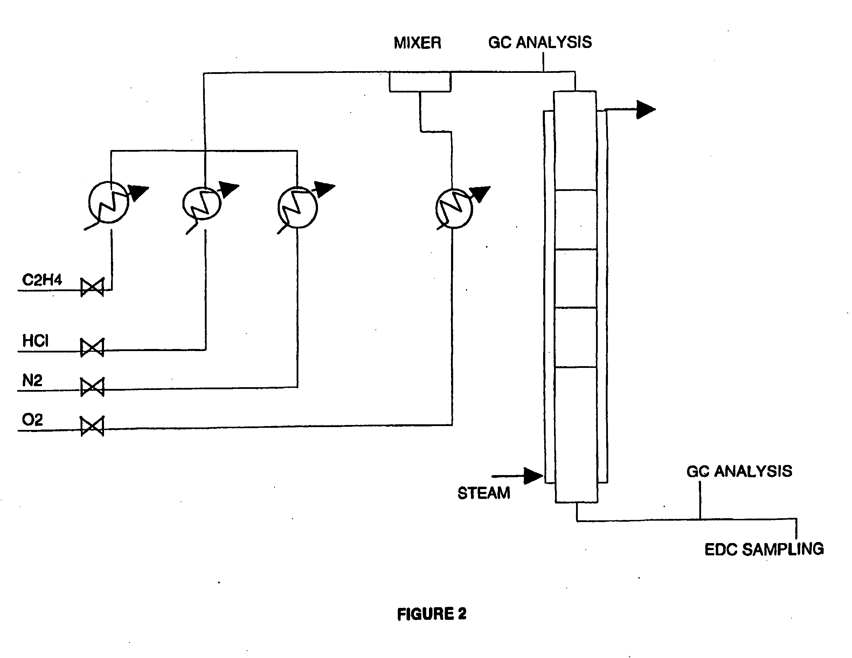

The apparatus used for the activity tests is schematically shown in Figure 2: it is a flow diagram illustrating the pilot plant for the catalytic oxychlorination of ethylene to 1,2-dichloroethane, which is used in the examples as representative of an exothermic reaction. The pilot reactor consisted of a single tube, which was loaded with the monolith catalyst samples before each test run. The reactors used were: two nickel tubes 9 m long with internal diameters of 27.2 mm (standard) or 56.0 mm (enlarged), and a nickel tube 6.5 m long with internal diameter of 56.0 mm. An external jacket with circulating boiling water was used to regulate the temperature profile. The reactor was equipped with one thermowell inserted into the central channel of the monoliths. A sliding thermocouple was used to record the temperature profile at different tube heights during the test. Two on-line gas chromatographs were used at the inlet and at the outlet of the reactor to control the reaction. The EDC (1, 2-dichloroethane) was collected in a vessel containing isopropyl alcohol at about 0°C and analyzed. This technique allows the collection also of the low boiling and water soluble compounds (chloroethanol, chloral, etc) as well as the unreacted HCl. The reactor feed was: 5200 dm³ STP/hour of ethylene, 600 dm³ STP / hour of oxygen, 2300 dm³ STP / hour of HCl, 1000 dm³ STP / hour of nitrogen. STP means standard temperature and pressure conditions, i.e. T = 0°C and P = 1 Ata (100 kPa).

The oxygen was 6.5 vol % (the flammability limit at 210 °C and 7 Ata (710 kPa) is about 8%). The inlet pressure of the reactor was 7 Ata (710 kPa), the inlet temperature was 150 °C and the temperature of the coolant was 240 °C in the case of metallic monoliths and 215°C in the case of the ceramic monolith.

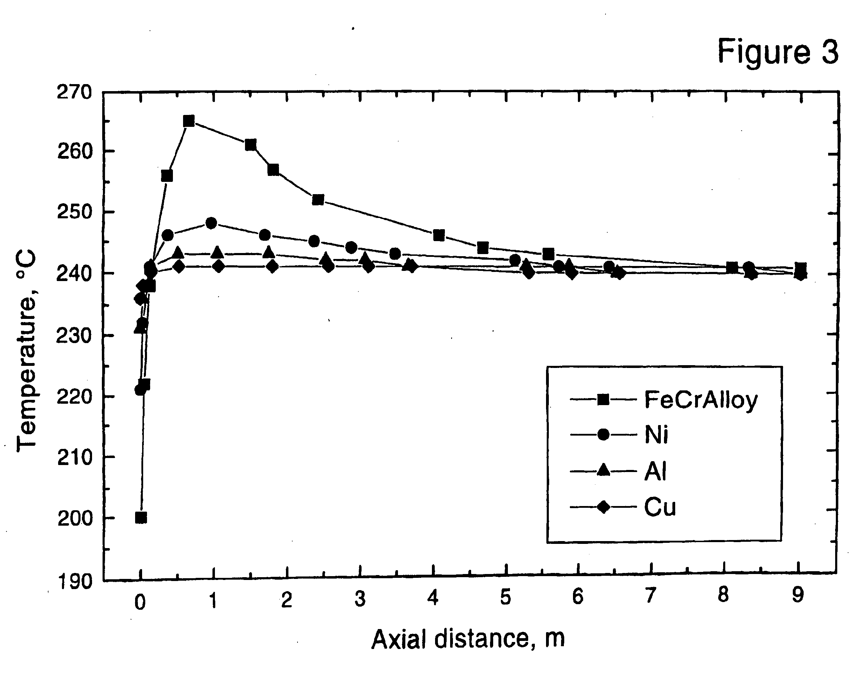

The results of the tests are reported in Table 1. The temperature profiles obtained in the most significant experiments are reported in Figures 3 to 6.

Figure 3 represents the temperature profiles obtained in test runs performed in the reactor having diameter = 27.2 mm and length = 9 m with catalysts supported, respectively, on Fecralloy®, nickel, aluminum and copper monoliths.

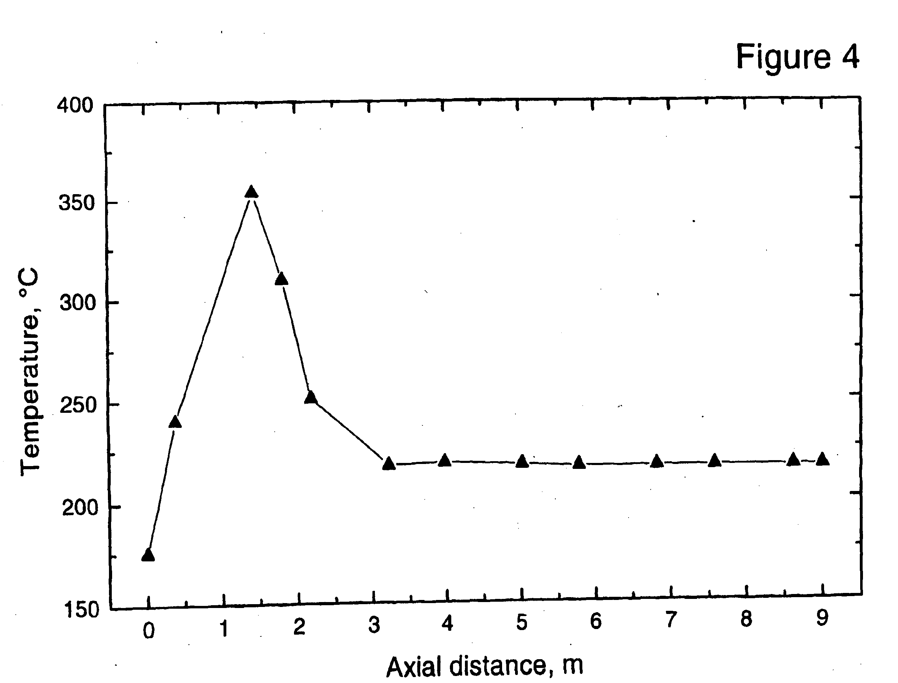

Figure 4 represents the temperature profile obtained in a test run performed in the reactor having diameter = 27.2 mm and length = 9 m with catalyst supported on cordierite monolith (comparative example).

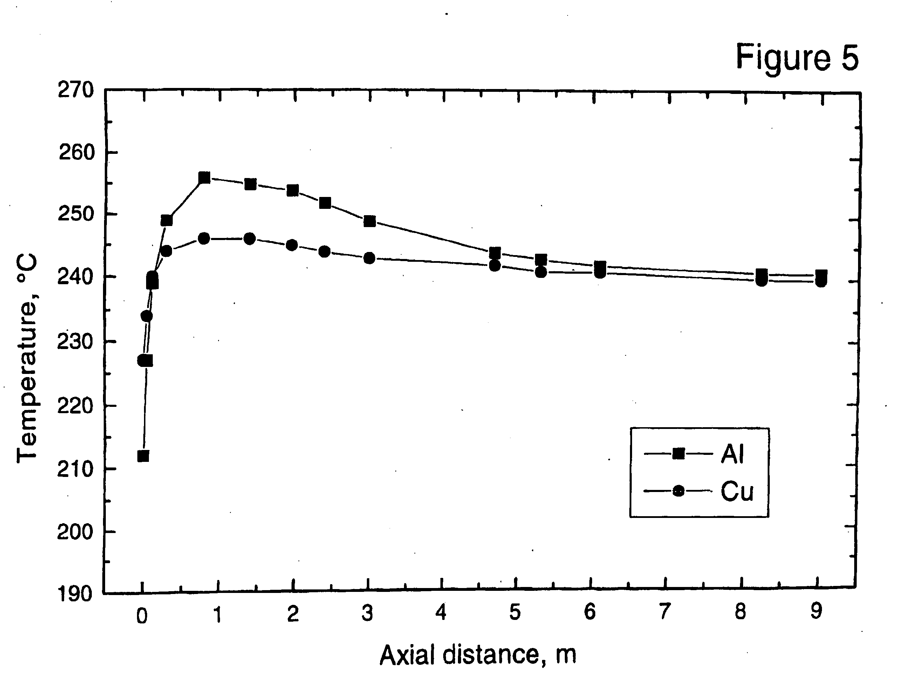

Figure 5 represents the temperature profiles obtained in test runs performed in the reactor having diameter = 56.0 mm and length = 9 m with catalyst supported, respectively, on aluminum and copper monoliths.

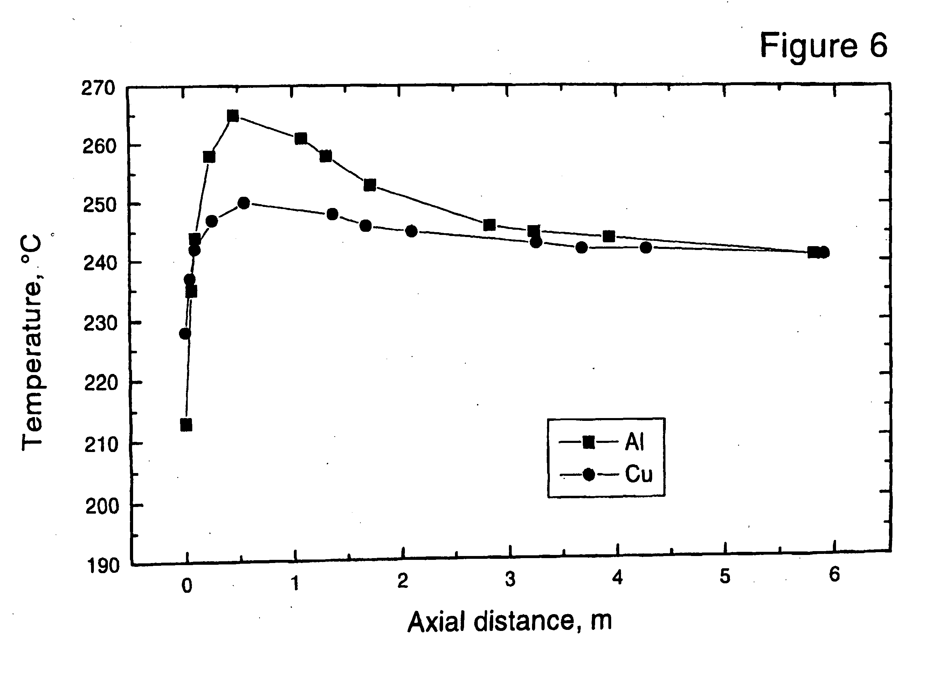

Figure 6 represents the temperature profiles obtained in test runs performed in the reactor having diameter = 56.0 mm and length = 6.5 m with catalyst supported, respectively, on aluminum and copper monoliths.

Under the adopted reaction conditions the O₂ and HCl conversions were always equal to or greater than 93.5 % and 99 %, respectively. The selectivity to 1,2-dichloroethane, obtained with metallic monoliths, was always greater than 98.5 % by moles, in some cases even greater than 99.5 % by moles. The selectivity to ethyl chloride, chlorinated by-products and to COₓ (x being an integer equal to 1 or 2), was always low, depending substantially on the hot spot temperature: the lower is the temperature, the lower is the formation of undesired by-products. On the contrary, the use of catalysts supported on ceramic monoliths did not allow a selectivity to 1,2-dichloroethane higher than 97 % by moles to be reached, because of the greater chlorinated by-products and COₓ formation. The difference in yield of 1.5 to 2 % by moles in the target products is very meaningful in industrial applications. The above results point out that the best comparison among the performances of different catalysts can be made by using the values of hot spot temperatures: the smaller is the difference between this value and the value of the coolant temperature, the better is the catalyst. From the reported results (see Table 1 and Figures 3 and 4) it is evident that all the catalysts based on metallic monoliths give better results than the catalyst supported on the ceramic one: the hot spot temperatures were lower than 270 °C in the present invention and higher than 350 °C in the comparison. The use of ceramic monoliths as a support gives place to hot spot temperatures too high to allow their use in an industrial reactor. On the contrary all the catalysts based on metallic monoliths give very flat temperature profiles in the standard industrial tubes (27.2 mm diameter). The difference with the coolant temperature is in the range from 2 °C to 17 °C. Among them, the best results were obtained using nickel, aluminum and copper monoliths.

Further it is well known to the skilled in the art that the lower the hot spot temperature, the longer the catalyst lifetime. Therefore the metallic monolith catalysts of the present invention show an improved lifetime with respect to the prior art catalysts.

On the basis of these results aluminum and copper based monoliths were loaded in tubes much larger than the standard ones (56 mm diameter). The obtained hot spot temperature was only a bit higher than that observed in smaller tubes and the difference with the coolant temperature was 18 °C in the case of aluminum and only 7 °C in the case of the copper (Figure 5).

The increase of washcoat layer thickness allowed the use of a shorter tube (6.5 m), owing to the increase of active components concentration per unit length of the tube. In spite of the increased reactivity, and thus of an increased heat production per unit length of the tube, the hot spot temperatures were only a bit higher than those observed in the longer tube with the same diameter: the difference was of 11 °C and, respectively, 4 °C using aluminum and copper based monoliths (Figure 6).

The above results prove the feasibility of adopting larger reactor tubes for industrial applications. It is well known to the skilled in the art that the use of such large reactor tubes loaded with conventional catalysts in pellets is not feasible because of very high hot spot temperatures, with the disadvantages indicated above. In order to reduce the hot spots with the conventional catalysts in pellets it is necessary to drastically reduce the productivity. This brings uneconomical industrial results.

The pressure drop, under the same productivity, in the examples of the invention was always very low, i.e. far below 5000 Pa (0.05 atm). In the comparative example using the ceramic monolith the pressure drop was significantly greater, although still below 1000 Pa (0.1 atm). With respect to the pellet catalyst the pressure drop with the monolith of the present invention is drastically reduced, being lower by about two orders of magnitude.

**TABLE 1**

| | Fecralloy | Nickel | Aluminum | Copper | Cordierite Cfr. | Aluminum | Copper | Aluminum | Copper |
|---|---|---|---|---|---|---|---|---|---|
| Tube diameter (mm) | 27.2 | 27.2 | 27.2 | 27.2 | 27.2 | 56.0 | 56.0 | 56.0 | 56.0 |
| Tube length (m) | 9.0 | 9.0 | 9.0 | 9.0 | 9.0 | 9.0 | 9.0 | 6.5 | 6.5 |
| Hot spot temperature (°C) | 267 | 248 | 244 | 242 | 355 | 258 | 247 | 269 | 251 |
| Difference between hot spot and coolant temperature (°C) | 27 | 8 | 4 | 2 | 140 | 18 | 17 | 29 | 11 |
| HCl conversion (%) | > 99.99 | 99.40 | 99.14 | 99.00 | 99.50 | 99.92 | 99.35 | 99.86 | 99.00 |
| Selectivity to 1,2-dichloroethane (mol%) | 98.53 | 98.72 | 98.79 | 98.86 | 96.80 | 98.65 | 98.77 | 98.58 | 98.70 |
| Selectivity to ethyl chloride (mol %). | 0.31 | 0.34 | 0.35 | 0.33 | 0.39 | 0.38 | 0.35 | 0.37 | 0.35 |
| Selectivity to chlorinated by-products (mol %) | 0.61 | 0.57 | 0.54 | 0.49 | 1.24 | 0.56 | 0.51 | 0.60 | 0.58 |
| Selectivity to COₓ(mol%) | 0.55 | 0.37 | 0.32 | 0.32 | 1.57 | 0.41 | 0.37 | 0.45 | 0.37 |

## Claims

1. Use of a catalyst comprising
a catalyst support that comprises a metallic monolith having channels the walls of which are adapted to receive a catalytically active phase or an intermediate layer acting as a carrier for a catalytically active phase;
wherein the intermediate layer is made of material selected from aluminum hydroxides, aluminum oxide-hydroxides, alumina, silica, zirconia, titania, magnesia, pumice, diatomaceous earth, zeolites and their mixtures; and
catalytically active material deposited on the walls of the channels, or optionally on the intermediate layer,
in a selective gas-phase exothermic reaction selected from the selective chlorination and/or oxychlorination of alkenes or alkanes or the selective oxidation of alkenes.

2. Use according to claim 1 wherein the channels are substantially parallel to the longitudinal axis of the monolith.

3. Use according to claim 1 or 2 wherein the perpendicular cross section of each channel forms a cell delimited by a closed line represented by the perimeter of the cross section of the channel walls.

4. Use according to any of claims 1 to 3 wherein the shape of each cell perimeter is regular.

5. Use according to claim 4, wherein said shape is square, triangular, hexagonal or circular.

6. Use according to any of claims 1 to 3 wherein the shape of each cell perimeter is irregular.

7. Use according to any of claims 1 to 6, wherein the cell density is at least 3 cells/cm².

8. Use according to claim 7 wherein the cell density is between 8 and 100 cells/cm².

9. Use according to any of claims 1 to 8 wherein the size of the cells is less than 5 mm.

10. Use according to claim 9 in which the size of the cells is between 1 and 3 mms.

11. Use according to any of claims 1 to 10 wherein the volume fraction of the metallic support is less than 0.9.

12. Use according to claim 11 wherein the volume fraction of the metallic support is between 0.15 and 0.6.

13. Use according to any of claims 1 to 12 wherein the surface area per unit volume of the monolith is at least 6 cm²/cm³.

14. Use according to claim 13 wherein the surface area per unit volume of the monolith is at least 10 cm²/cm³.

15. Use according to any of claims 1 to 14, wherein the length of the monolith is at least 5 cms.

16. Use according to claim 15 wherein the length of the monolith is in the range 30 cms to 1 m.

17. Use according to any of claims 1 to 16 wherein the metallic structure formed by the channel walls of the monolith is continuous.

18. Use according to any of claims 1 to 17, made of a metal chosen from copper, aluminum, nickel and alloys thereof.

19. Use according to any of claims 1 to 17, made of an alloy comprising predominantly iron, chromium and aluminum.

20. Use according to any of claims 1 to 19 wherein the surface of the monolith is covered by an intermediate layer acting as a carrier for a catalytically active compound.

21. Use according to claim 1, wherein the catalyst fills a tubular reactor and the walls of the monoliths are in contact with the wall of the reactor.

22. Use according to any one of claims 1 to 21, wherein the reaction is selected from the conversion of ethylene with chlorine to 1,2-dichloroethane; the conversion of ethylene with hydrogen chloride with air or oxygen to give 1,2-dichloroethane; the conversion of ethane with hydrogen chloride with air or oxygen to give a saturated or unsaturated chlorinated hydrocarbon, preferably 1,2-dichloroethane or vinyl chloride; and the reaction of methane with chlorine.

23. Use according to claim22, wherein the catalyst for the oxychlorination reaction of ethylene contains copper in an amount of 1 to 12 wt % of the intermediate layer.

24. Use according to claim 23, wherein the catalyst also comprises at least one alkali metal, alkaline earth metal, group IIB metal or lanthanide in a total amount up to 6 wt% of the intermediate layer.

25. Use according to claim 22, wherein the catalyst for the oxychlorination reaction of ethane contains in the intermediate layer copper and an alkali metal in the atomic ratio 2:8.

26. Use according to claim 25, wherein the catalyst also comprises at least one alkaline earth metal, group IIB metal or lanthanide.

27. Use according to claim 1, wherein the catalyst for the selective oxidation reaction of ethylene comprises at least silver, and at least one alkali and/or alkaline earth metal.

## Patentansprüche

1. Verwendung eines Katalysators, umfassend
einen Katalysatorträger, der einen metallischen Monolith mit Kanälen umfaßt, dessen Wände so angepaßt sind, daß sie eine katalytisch aktive Phase oder eine Zwischenschicht, die als Träger für die katalytisch aktive Phase fungiert, aufnehmen;
wobei die Zwischenschicht aus einem Material ist, ausgewählt aus Aluminiumhydroxiden, Aluminiumoxidhydroxiden, Aluminiumoxid, Siliciumdioxid, Zirkoniumdioxid, Titandioxid, Magnesiumoxid, Bimsstein, Diatomeenerde, Zeolithen und ihren Gemischen; und
katalytisch aktives Material, das auf den Wänden der Kanäle oder gegebenenfalls auf der Zwischenschicht abgeschieden ist,
in einer selektiven exothermen Gasphasenreaktion, ausgewählt aus selektiver Chlorierung und/oder Oxychlorierung von Alkenen oder Alkanen oder selektiver Oxidation von Alkenen.

2. Verwendung nach Anspruch 1, wobei die Kanäle im wesentlichen parallel zu der Längsachse des Monolithen angeordnet sind.

3. Verwendung nach Anspruch 1 oder 2, wobei der senkrechte Querschnitt von jedem Kanal eine Zelle bildet, die durch eine geschlossene Linie, die den Umfang des Querschnittes der Kanalwände darstellt, abgegrenzt ist.

4. Verwendung nach einem der Ansprüche 1 bis 3, wobei die Form jedes Zellumfangs regelmäßig ist.

5. Verwendung nach Anspruch 4, wobei die Form quadratisch, dreieckig, hexagonal oder kreisförmig ist.

6. Verwendung nach einem der Ansprüche 1 bis 3, wobei die Form jedes Zellumfangs unregelmäßig ist.

7. Verwendung nach einem der Ansprüche 1 bis 6, wobei die Zelldichte mindestens 3 Zellen/cm² beträgt.

8. Verwendung nach Anspruch 7, wobei die Zelldichte zwischen 8 und 100 Zellen/cm² liegt.

9. Verwendung nach einem der Ansprüche 1 bis 8, wobei die Größe der Zellen weniger als 5 mm beträgt.

10. Verwendung nach Anspruch 9, wobei die Größe der Zellen zwischen 1 und 3 mm liegt.

11. Verwendung nach einem der Ansprüche 1 bis 10, wobei die Volumenfraktion des metallischen Trägers weniger als 0,9 beträgt.

12. Verwendung nach Anspruch 11, wobei die Volumenfraktion des metallischen Trägers zwischen 0,15 und 0,6 liegt.

13. Verwendung nach einem der Ansprüche 1 bis 12, wobei die Oberfläche pro Volumeneinheit des Monolithen mindestens 6 cm²/cm³ beträgt.

14. Verwendung nach Anspruch 13, wobei die Oberfläche pro Volumeneinheit des Monolithen mindestens 10 cm²/cm³ beträgt.

15. Verwendung nach einem der Ansprüche 1 bis 14, wobei die Länge des Monolithen mindestens 5 cm beträgt.

16. Verwendung nach Anspruch 15, wobei die Länge des Monolithen in dem Bereich von 30 cm bis 1 m liegt.

17. Verwendung nach einem der Ansprüche 1 bis 16, wobei die metallische Struktur, die von den Kanalwänden des Monolithen gebildet wird, kontinuierlich ist.

18. Verwendung nach einem der Ansprüche 1 bis 17, hergestellt aus einem Metall, ausgewählt aus Kupfer, Aluminium, Nickel und Legierungen davon.

19. Verwendung nach einem der Ansprüche 1 bis 17, hergestellt aus einer Legierung, überwiegend umfassend Eisen, Chrom und Aluminium.

20. Verwendung nach einem der Ansprüche 1 bis 19, wobei die Oberfläche des Monolithen von einer Zwischenschicht, die als Träger für eine katalytisch aktive Verbindung fungiert, bedeckt ist.

21. Verwendung nach Anspruch 1, wobei der Katalysator einen Rohrreaktor füllt und die Wände der Monolithen mit der Wand des Reaktors in Kontakt stehen.

22. Verwendung nach einem der Ansprüche 1 bis 21, wobei die Reaktion aus der Umwandlung von Ethylen mit Chlor zu 1,2-Dichlorethan; der Umwandlung von Ethylen mit Hydrogenchlorid mit Luft oder Sauerstoff unter Erhalt von 1,2-Dichlorethan; der Umwandlung von Ethan mit Hydrogenchlorid mit Luft oder Sauerstoff unter Erhalt eines gesättigten oder ungesättigten chlorierten Kohlenwasserstoffes, bevorzugt 1,2-Dichlorethan oder Vinylchlorid; und der Reaktion von Methan mit Chlor ausgewählt ist.

23. Verwendung nach Anspruch 22, wobei der Katalysator für die Oxychlorierungsreaktion von Ethylen Kupfer in einer Menge von 1 bis 12 Gew.-% der Zwischenschicht enthält.

24. Verwendung nach Anspruch 23, wobei der Katalysator ebenso mindestens ein Alkalimetall, Erdalkalimetall, Metall der Gruppe IIB oder Lanthanoid in einer Gesamtmenge von bis zu 6 Gew.-% der Zwischenschicht umfaßt.

25. Verwendung nach Anspruch 22, wobei der Katalysator für die Oxychlorierungsreaktion von Ethan in der Zwischenschicht Kupfer und ein Alkalimetall in dem Atomverhältnis 2 : 8 enthält.

26. Verwendung nach Anspruch 25, wobei der Katalysator ebenso mindestens ein Erdalkalimetall, Metall der Gruppe IIB oder Lanthanoid umfaßt.

27. Verwendung nach Anspruch 1, wobei der Katalysator für die selektive Oxidationsreaktion von Ethylen zumindest Silber und zumindest ein Alkali- und/oder Erdalkalimetall umfaßt.

## Revendications

1. Utilisation d'un catalyseur comprenant
un support de catalyseur qui comprend un monolithe métallique ayant des canaux dont les parois sont adaptées pour recevoir une phase catalytiquement active ou une couche intermédiaire servant de support pour une phase catalytiquement active ;
dans laquelle la couche intermédiaire est constituée d'un matériau choisi parmi les hydroxydes d'aluminium, les oxydes-hydroxydes d'aluminium, l'alumine, la silice, la zircone, l'oxyde de titane, la magnésie, la ponce, la terre de diatomée, les zéolites et leurs mélanges ; et
un matériau catalytiquement actif déposé sur les parois des canaux ou facultativement sur la couche intermédiaire,
dans une réaction exothermique en phase gazeuse choisie parmi la chloration et/ou l'oxychloration d'alcènes ou d'alcanes ou l'oxydation sélective d'alcènes.

2. Utilisation selon la revendication 1, dans laquelle les canaux sont sensiblement parallèles à l'axe longitudinal du monolithe.

3. Utilisation selon la revendication 1 ou 2, dans laquelle la coupe transversale perpendiculaire de chaque canal forme une cellule délimitée par une ligne fermée représentée par le périmètre de la coupe transversale des parois du canal.

4. Utilisation selon l'une quelconque des revendications 1 à 3, dans laquelle la forme du périmètre de chaque cellule est régulière.

5. Utilisation selon la revendication 4, dans laquelle ladite forme est carrée, triangulaire, hexagonale ou circulaire.

6. Utilisation selon l'une quelconque des revendications 1 à 3, dans laquelle la forme du périmètre de chaque cellule est irrégulière.

7. Utilisation selon l'une quelconque des revendications 1 à 6, dans laquelle la densité de cellule est d'au moins 3 cellules/cm².

8. Utilisation selon la revendication 7, dans laquelle la densité de cellule est comprise entre 8 et 100 cellules/cm².

9. Utilisation selon l'une quelconque des revendications 1 à 8, dans laquelle la taille des cellules est inférieure à 5 mm.

10. Utilisation selon la revendication 9, dans laquelle la taille des cellules est comprise entre 1 et 3 mm.

11. Utilisation selon l'une quelconque des revendications 1 à 10, dans laquelle la fraction en volume du support métallique est inférieure à 0,9.

12. Utilisation selon la revendication 11, dans laquelle la fraction en volume du support métallique est comprise entre 0,15 et 0,6.

13. Utilisation selon l'une quelconque des revendications 1 à 12, dans laquelle la surface spécifique par unité de volume du monolithe est d'au moins 6 cm²/cm³.

14. Utilisation selon la revendication 13, dans laquelle la surface spécifique par unité de volume du monolithe est d'au moins 10 cm²/cm³.

15. Utilisation selon l'une quelconque des revendications 1 à 14, dans laquelle la longueur du monolithe est d'au moins 5 cm.

16. Utilisation selon la revendication 15, dans laquelle la longueur du monolithe est comprise dans la plage allant de 30 cm à 1 m.

17. Utilisation selon l'une quelconque des revendications 1 à 16, dans laquelle la structure métallique formée par les parois de canaux du monolithe est continue.

18. Utilisation selon l'une quelconque des revendications 1 à 17, constituée d'un métal choisi parmi le cuivre, l'aluminium, le nickel et leurs alliages.

19. Utilisation selon l'une quelconque des revendications 1 à 17, constituée d'un alliage comprenant principalement du fer, du chrome et de l'aluminium.

20. Utilisation selon l'une quelconque des revendications 1 à 19, dans laquelle la surface du monolithe est couverte par une couche intermédiaire servant de support pour un composé catalytiquement actif.

21. Utilisation selon la revendication 1, dans laquelle le catalyseur remplit un réacteur tubulaire et les parois des monolithes sont en contact avec la paroi du réacteur.

22. Utilisation selon l'une quelconque des revendications 1 à 21, dans laquelle la réaction est choisie parmi la conversion de l'éthylène avec du chlore en 1,2-dichloroéthane ; la conversion de l'éthylène avec du chlorure d'hydrogène avec de l'air ou de l'oxygène pour donner du 1,2-dichloroéthane ; la conversion de l'éthane avec du chlorure d'hydrogène avec de l'air ou de l'oxygène pour donner un hydrocarbure chloré saturé ou insaturé, de préférence le 1,2-dichloroéthane ou le chlorure de vinyle ; et la réaction du méthane avec le chlore.

23. Utilisation selon la revendication 22, dans laquelle le catalyseur pour la réaction d'oxychloration de l'éthylène contient du cuivre en une quantité de 1 à 12 % en poids de la couche intermédiaire.

24. Utilisation selon la revendication 23, dans laquelle le catalyseur comprend également au moins un métal alcalin, un métal alcalino-terreux, un métal du groupe IIB ou un lanthanide en une quantité totale allant jusqu'à 6 % en poids de la couche intermédiaire.

25. Utilisation selon la revendication 22, dans laquelle le catalyseur pour la réaction d'oxychloration de l'éthane contient dans la couche intermédiaire du cuivre et un métal alcalin dans le rapport atomique de 2 : 8.

26. Utilisation selon la revendication 25, dans laquelle le catalyseur comprend également au moins un métal alcalino-terreux, un métal du groupe IIB ou un lanthanide.

27. Utilisation selon la revendication 1, dans laquelle le catalyseur pour la réaction d'oxydation sélective de l'éthylène comprend au moins de l'argent et au moins un métal alcalin et/ou alcalino-terreux.
